Europäisches Patentamt

**European Patent Office**

Office européen des brevets

(11) Numéro de publication: **0 096 731**
**A1**

## (12) DEMANDE DE BREVET EUROPEEN

(21) Numéro de dépôt: **82810245.9**

(22) Date de dépôt: **09.06.82**

(51) Int. Cl.³: **A 61 N 1/04,** A 61 M 11/00, B 05 B 1/00

(43) Date de publication de la demande: **28.12.83**
**Bulletin 83/52**

(71) Demandeur: **Bernaz, Gabriel, 35, rue E. Marziano, CH-1227 Carouge (CH)**

(72) Inventeur: **Bernaz, Gabriel, 35, rue E. Marziano, CH-1227 Carouge (CH)**

(84) Etats contractants désignés: **AT BE CH DE FR GB IT LI LU NL SE**

(74) Mandataire: **Mohnhaupt, Dietrich et al, DIETLIN, MOHNHAUPT & CIE 3, quai du Mont-Blanc, CH-1201 Genève (CH)**

(54) **Appareil pour soins esthétiques et électrothérapeutiques et dispositif d'atomisation.**

(57) L'appareil est constitué d'une baie d'alimentation et d'une poignée de distribution (1) reliée à la baie par une conduite multifonction flexible, via un raccord rapide. La poignée est formée d'un corps (2), d'une tête (4) et d'un capuchon (3), le corps et la tête étant d'une pièce. La tête (4) forme une cuvette (5) dans laquelle est logée au moins une électrode (7), une éponge torique (6) et, centralement, un dispositif d'atomisation (10). Celui-ci comprend une chambre de prémélange (20), au moins un canal longitudinal (18), une chambre antérieure de turbulence (21) et une ouverture centrale à bords francs (17).

ACTORUM AG

## Appareil pour soins esthétiques et électrothérapeutiques et dispositif d'atomisation

L'invention a pour objet un appareil pour soins esthétiques et électrothérapeutiques par traitement cutané et transcutané. Elle concerne également un dispositif d'atomisation ou de pulvérisation d'un liquide.

Le résultat des soins donnés par l'appareil est en majeure partie dû à des effets physico-chimiques et électrodynamiques créés par un courant électrique appliqué à la peau. Il s'ensuit une réaction chimico-cutanée.

Le brevet suisse no. 625 659 décrit un dispositif pour soins esthétiques et électrothérapeutiques comprenant au moins une électrode et une poignée distincte de cette électrode, la poignée et l'électrode comportant des moyens d'accouplement pour fixer l'électrode de façon amovible à la poignée, et à permettre à la poignée de fournir à l'électrode une tension et/ou un courant et au moins un fluide selon les besoins.

L'accouplement de l'électrode à la poignée se fait, selon ce brevet, par un raccord rapide, les connexions

- 2 -                                0096731

électriques étant réalisées par une ou plusieurs fiches
qui doivent être insérées dans des prises ou douilles
correspondantes de la poignée.  Simultanément, une cavité
est formée dans laquelle un fluide gazeux, normalement de
l'air, et un fluide liquide, en général de l'eau, peuvent
se mélanger pour former un spray lorsque le mélange formé
passe par une tuyère pratiquée centralement au fond de
la pièce porte-électrode.  Cette pièce comporte en plus
un doigt de positionnement qui doit être inséré dans un
trou borgne de la poignée.

On a constaté que cette construction de la
poignée comporte un nombre de désavantages.

D'abord, l'utilisateur trouve souvent difficile de positionner la tête porte-électrode correctement
par rapport à la poignée pour raccorder ces deux organes.
Le doigt de positionnement ainsi que les fiches de
connexion électrique peuvent être faussés et n'entrent
alors plus dans leurs trous ou douilles situés dans la
poignée.  En plus, la formation et la qualité du spray
laissait beaucoup à désirer, en partie dû au fait que la
forme de la cavité mélangeuse est différente si la tête
porte-électrode était poussée à fond dans la poignée ou
non.

L'invention vise à supprimer ou a atténuer ces
inconvénients.  Elle a pour but de proposer une nouvelle
construction plus sûre et plus solide de la poignée, plus
facilement à manipuler lors de l'usage.  Un autre but
de l'invention est de développer un nouveau dispositif
d'atomisation apte à donner un spray parfait, a gouttelettes ultra-fines, ne nécessitant qu'un minimum d'air
de suspension et de propulsion.  En effet, le spray a prin-

- 3 -                    0096731

cipalement la fonction de mouiller les électrodes et la peau; le liquide est généralement une lotion aqueuse comportant des substances de traitement cosmétique ou pharmaceutique de la peau. L'air est uniquement nécessaire pour former un spray correct et, le cas échéant, pour véhiculer de l'ozone. Un excès d'air est donc inutile et à éviter. Les constructions d'atomiseurs connues ne pouvaient pas remplir ces buts.

Le dispositif d'atomisation et l'appareil selon l'invention sont définis dans les revendications indépendantes tandis que des réalisations particulières font l'objet des revendications dépendantes.

L'invention sera expliquée plus en détail par la description suivante de modes de réalisation, donnés à titre d'exemple non-limitatif du dispositif et de l'appareil selon l'invention, et à l'aide du dessin dans lequel :

> la fig. 1    est une coupe axiale d'une poignée faisant partie de l'appareil selon l'invention,
>
> la fig. 2    est une coupe transversale selon la ligne II-II de la fig. 1,
>
> la fig. 3    est une coupe axiale du dispositif d'atomisation selon l'invention, à l'état dévissé,
>
> la fig. 4    est une coupe axiale de la partie frontale d'une autre poignée, et
>
> la fig. 5    montre le schéma-bloc de l'appareil selon l'invention.

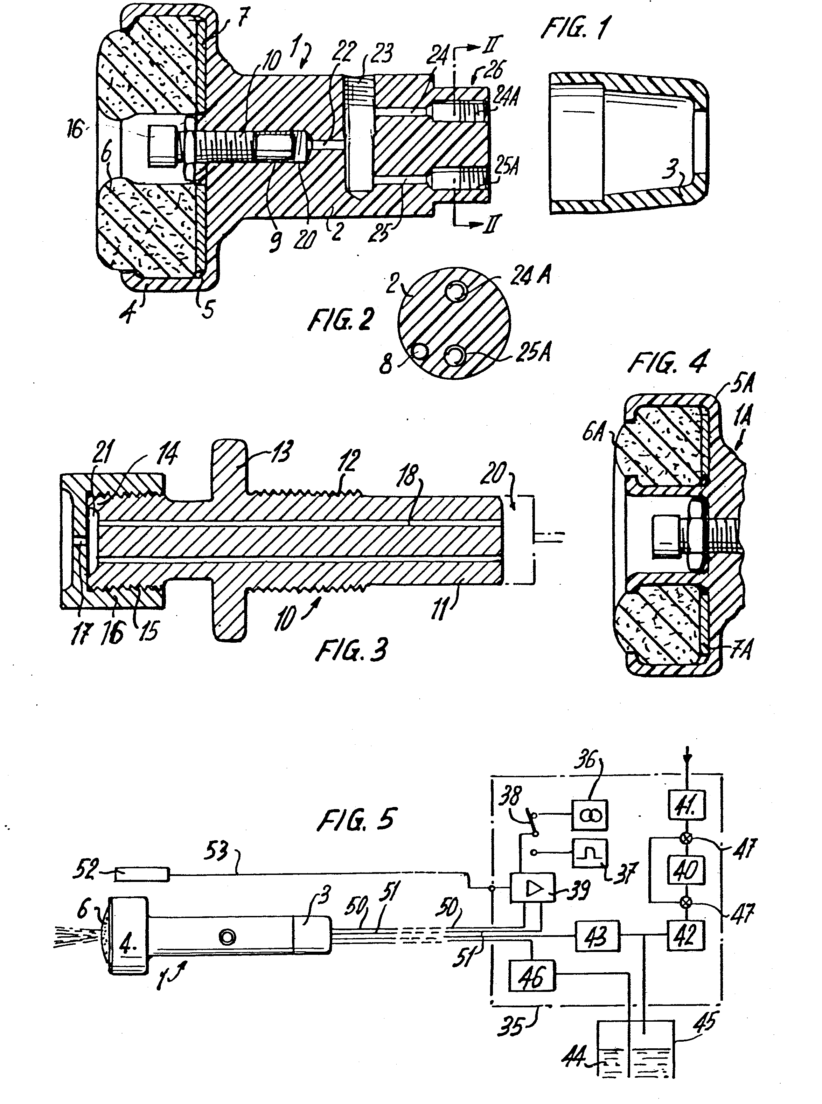

La poignée 1 représentée à la fig. 1 est de

section extérieure ronde et est fabriquée de préférence
en matière synthétique isolante, moulable, dure et
ayant une bonne stabilité dimensionnelle, par exemple
nylon, polychlorure de vinyle ou de vinylidène dur, y
compris les copolymères, polycarbonate, polyacétale etc.
Le corps principal 2 comporte, à la partie frontale, une
tête 4 formée intégralement au corps 2. Cette tête 4
comprend une cuvette 5, à rebord rentrant périphérique
frontal, portant une éponge annulaire 6 à pores ouvertes
qui s'appuie contre les parois latérales de la tête 2
et, au fond de la cuvette 5, contre une rondelle 7, en
matériau conducteur de l'électricité et resistant à la
corrosion. Un fil non représenté, relié solidement par
derrière à la rondelle 7, passe par un canal 8 (voir
fig. 2) pratiqué dans le corps 2 de la poignée et relie
la rondelle 7 et, par contact, l'éponge 6 électriquement
au boîtier de l'appareil comme il sera décrit plus bas.
La rondelle peut être réalisée en un alliage plomb-étain,
en acier inoxydable ou encore en un matériau conducteur
peu résistant à la corrosion mais revêtu d'une couche
résistante.

Le volume et les dimensions de l'éponge 6 sont
choisis pour qu'elle déborde frontalement de la cuvette 5
tout en étant retenue par le rebord périphérique.

La poignée 1 comporte une alésage central 9
dans lequel est vissé le dispositif d'atomisation 10 qui
sera décrit en détail plus bas. La longueur du dispositif
10 et de l'alésage 9 sont choisis pour former une
chambre de prémélange 20 au fond de l'alésage. L'alésage
9 se prolonge axialement par un canal circulaire 22
jusqu'à un trou latéral borgne 23 servant à recevoir un
robinet-poussoir qui commande l'admission et l'arrêt des fluides formant le spray.

Les canaux 24 et 25 sont percés dans la partie
postérieure du corps 2 et se terminent dans le trou borgne
23, d'une part, et dans la surface arrière par un alésage
fileté 24A, 25A d'autre part. Ces alésages 24A, 25A
servent à recevoir un raccord vissé avec un tuyaux flexible
(non représenté) qui mène au boîtier de l'appareil. Les
canaux 24 et 25 sont en général symétriques par rapport
à un plan vertical comprenant l'axe de la poignée, et le
canal 24 est prévu pour l'eau et le canal 25 pour l'air.

Finalement, ces raccords d'eau, d'air et
d'électricité sont couverts et protégés par le capot 3
qui s'emboîte sur la partie postérieure de la poignée
comportant un rétrécissement 26.

On voit que la poignée 1 est d'une construction
en monobloc solide, rigide et robuste, et qu'il n'y a
pas de pièces qui doivent être raccordées ou emboîtées,
voire positionnées, par l'utilisateur qui veut se servir
de l'appareil.

La fig. 3 montre le dispositif d'atomisation
selon l'invention qui est utilisé avec l'appareil qui
vient d'être décrit mais qui peu servir, indépendamment
de celui-ci, à toutes les utilisations où l'on doit
produire un spray ultra-fin, régulier et ne demandant
que le minimum d'air comme fluide de propulsion. Il
peut notamment être utilisé dans toutes les bombes à
aérosol connues.

Ce dispositif 10 comprend un corps principal
cylindrique 11, agencée pour être vissé dans un alesage au moyen d'un filetage extérieur 12 et d'un écrou 13;
cependant, ces moyens de fixation peuvent être remplacés
par l'autres équivalents, selon le cas particulier;
l'homme du métier est familier avec ces fixations.

La partie antérieure frontale du corps 11, à
gauche dans la fig. 3, comporte un rebord circulare 14
d'une hauteur d'environ 0,1 à 0,2 mm, typiquement 0,11 mm,
et un filetage extérieur 15.  Un capuchon 16 est vissée sur
ce filetage 15 jusqu'a ce que sa face intérieure plane
entre en contact avec le bord antérieur du rebord 14.
Il se forme donc à l'intérieur du capuchon 16 une chambre
de turbulence 21.  Une ouverture 17, d'un diamètre adapté
au débit du spray désiré, est pratiquée dans la paroi
frontale du capuchon 16.  Son diamètre qui détermine
la forme et le débit du spray, est par exemple de 0,35 mm,
pour l'utilisation visée en particulier ici.  Cependant,
par un essai simple et rapide, l'homme du métier peut
trouver un autre diamètre adapté à un autre débit et/ou
à d'autres dimensions du dispositif.

Il est très important que les bords circulaires
de l'ouverture, en particulier le bord interne, soient
francs et nets et que la paroi cylindrique de l'ouverture
forme un angle de 90$^{\circ}$ avec les deux faces parallèles
planes du capuchon, c'est-à-dire que l'ouverture est
cylindrique dans l'axe du dispositif et sans bord arrondi
ou arêtes.  Ces conditions sont importantes pour l'atomisation du liquide.

Un, deux ou plusieurs canaux 18 relient la chambre
de turbulence à l'extrémité arrière du corps 11.  Ils
servent au passage d'un prémélange de liquide et d'air
provenant d'une chambre de prémélange 20 qui se trouve derrière le dispositif 10, voir la fig. 1. Le diamètre et le nombre des canaux 18 sont tels qu'une différence de pression est créée entre les deux chambres 20 et 21. Dans l'exécution représentée, il y a deux canaux 18 ayant chacun un diamètre de 0,8 mm. Mais un canal suffit en principe; il ne doit pas être situé dans l'axe du dispositif.

Lors de l'utilisation pour former un spray, la chambre 20 est alimentée, selon la fig. 1, par le canal 22, en air et en eau. La pression de l'eau et de l'air qui entrent dans les alésages 24A et 25A, respectivement, peut atteindre quelques bars; dans l'exemple choisi, elle est de 0,4 bar environ. Un premier mélange, dans un courant plus ou moins laminaire, d'eau et d'air se fait dans le canal circulaire 22 et dans la chambre de prémélange 20. Le mélange final est effectuée à l'état turbulent dans la chambre 21, et un spray parfaitement stable, aux gouttelettes extrêmement fines et régulières, sort de l'orifice 17. L'angle d'ouverture du spray conique est de 5 à 30$^O$, selon le rapport longueur: diamètre de l'ouverture 17. Le débit en eau est d'environ 15 à 20 ml/min, le débit en air est très faible et probablement inférieur à 100 ml/min.

Le dispositif d'atomisation est vissé par devant dans alésage 9 de la poignée 1. Il peut donc être facilement enlevé et nettoyé ou remplacé.

L'éponge 6 est imbibée par le liquide du spray pour assurer la conductibilité entre le disque 7 et la peau d'un patient sur laquelle la poignée est appliquée.

La fig. 4 montre, par une coupe arrachée axiale de la partie frontale d'une autre poignée 1A, l'utilisation d'une électrode multiple. La cuvette 5 de la fig. 1 est subdivisée par des parois verticales en deux, trois
ou quatre cuvettes 5A à rebord rentrant comprenant
chacune une éponge cylindrique 6A et une électrode
métallique plate 7A; chaque électrode est reliée par un
fil (non représenté) avec le boîtier de l'appareil
qui sera décrit plus bas. Cette configuration permet de
faire passer un courant entre deux ou plusieurs pôles 7A
des cuvettes 5A à travers les éponges 6A imbibées de
liquide, et la peau d'un patient. Plusieurs tensions
différentes peuvent alors être appliquées.

La fig. 5 représente un schéma fonctionnel
de l'appareil selon l'invention.

Une baie 35 comprend une source de courant
continu constant 36 et un générateur d'impulsions 37.
Ces impulsions ont normalement une forme rectangulaire;
l'appareil permet de régler et bien le rapport cyclique
des impulsions et bien leur séquence, c'est-à-dire on
peut faire alterner un train d'impulsions avec un arrêt
d'impulsions. En plus, la fréquence des impulsions peut
être réglable indépendamment du rapport cyclique.

Un commutateur 38 permet de choisir ces
courants et de les appliquer à l'entrée d'un amplificateur
39 dont la sortie et reliée aux fils 50 et 51. La baie
35 comprend en outre une électropompe 40 aspirant de l'air
à travers un filtre 41. L'air comprimé passe par un générateur d'ozone 42 et d'un corps de chauffe 43 dont la
sortie est reliée à l'un des tuyaux souples alimentant
la poignée 1.

La pression de l'air s'exerce également au-
dessus de la surface d'un liquide ou lotion aqueuse 44 qui se trouve dans un récipient 45. Le liquide sous pression passe à travers un régulateur de débit 46 dans l'autre tuyau souple d'alimentation de la poignée 1. Le débit de l'air qui entre peut également être réglé par un régulateur.

La poignée est raccordée à la baie 35 par un raccord rapide connu en soi, non représenté.

Des robinets 47 ou électrovalves permettent dans certains cas de court-circuiter l'électropompe 40, une bouteille d'oxygène ou d'air sous pression peut alors alimenter l'appareil.

Lors l'utilisation de l'appareil, le patient touche une électrode de retour, appelée électrode inactive 52, reliée à l'amplificateur 39 dans la baie 35 au moyen du fil 53.

La tension appliquée au patient est donc flot-tante, à savoir le niveau zéro peut changer selon les caractéristiques bioélectriques du patient.

Grâce à l'appareil décrit et représenté on peut prodiguer un vaste éventail de soins avec une seule ou plusieurs poignées de distribution coopérant avec un certain nombre d'électrodes, et une seule baie, les poignées étant rapidement interchangeables. Pour chaque électrode on dispose de toutes les rassources des moyens électromécaniques. Par exemple, on peut utiliser l'électrode monopolaire de manière à délivrer simultanément des impulsions et un spray, ou on peut utiliser une électrode biopolaire de manière à délivrer un courant continu et un spray.

Outre la ficilité de manipulation et la diminution
de l'encombrement, le dispositif décrit et représenté
est d'un coût relativement faible.  En effet, en groupant
plusieurs fonctions électriques on n'a, par exemple,
besoin que d'une alimentation.

L'appareil selon l'invention peut être modifié
dans le cadre de ce qui est revendiqué.  Par exemple, la
forme, la fréquence et l'amplitude du courant appliqué
par la poignée au patient peuvent être différentes de
celle décrites ici.  En outre, la forme de la poignée
peut être adaptée aux besoins des soins ou traitements
envisagés.

Une modification préférée consiste en la réalisation d'un système d'aspiration du liquide appliqué en
spray sur le peau du patient et sur les éponges.  En
effet, il peut être gênant lorsqu'il y a un excès de
liquide sur la peau.

Ce système qui n'est pas représenté sur le
dessin car il peut facilement être réalisé par l'homme
du métier, comprend un canal traversant la poignée dans
le sens de la longueur et aboutissant dans la cuvette 5.
Ce canal est relié par un tuyau flixible à une deuxième
tête de l'électropompe, à travers un récipent de
séparation.  L'électropompe peut donc aspirer le liquide
en trop et le récupérer.  Cette aspiration peut être
enclenchée en continu ou de façon intermittente.

REVENDICATIONS

1. - Appareil pour soins esthétiques et électrothérapeutiques, comprenant une poignée pour la distribution
d'une tension électrique et d'un spray, caractérisé en ce
que la poignée comporte intégralement une cuvette dans
laquelle est montée au moins une électrode active et une
éponge pour transmettre la tension de l'électrode à la
peau d'un patient, et au centre un dispositif d'atomisation d'un liquide, ce dispositif d'atomisation étant
entouré par l'électrode ou les électrodes, la poignée étant
reliée par une conduite flexible à une baie renfermant
l'alimentation en courant en fluide.

2. - Appareil selon la revendication 1, caractérisé en ce le dispositif d'atomisation est vissé dans
un alésage central de la poignée de façon à former, à
l'intérieur de l'alésage qui est relié au fond par un
canal à la cavité d'une soupape contrôlant l'admission
d'un gaz et d'un liquide aqueux sous pression, une chambre
de prémélange des fluides.

3. - Appareil selon la revendication 1 ou 2,
caractérisé en ce que la conduite flexible reliant la
poignée à la baie, comprend des canaux pour un gaz sous
pression, un liquide aqueux sous pression, et, pour
chaque électrode active, un câble d'alimentation.

4. - Appareil selon la revendication 3, caractérisé en ce que la poignée comporte également un canal
d'évacuation de liquide traversant la poignée en longueur
entre la cuvette et l'autre extrémité, la dite conduite
flexible comprenant en plus un canal d'évacuation relié
dans la baie de l'appareil à la deuxième tête d'une électropompe, la première tête servant à créer les pressions hydraulique et pneumatique.

5. - Appareil selon l'une des revendications précédentes, caractérisés en ce qu'il comprend un ensemble de plusieurs électrodes distinctes dans la cuvette, chaque électrode étant reliée séparément à une source de tension dans la baie.

6. - Appareil selon l'une des revendications précédentes, caractérisé en ce que la conduite flexible est raccordée à la baie par un raccord rapide, différentes poignées pouvant alors être accouplées à l'appareil.

7. - Appareil selon l'une des revendications précédentes, caractérisé en ce que le dispositif d'atomisation est alimenté par de l'air ou de l'oxygène et de l'eau ou d'une lotion aqueuse, les deux fluides étant sous pression d'environ 0,4 bar.

8. - Appareil selon l'une des revendications précédentes, caractérisé en ce qu'il comprend un générateur d'ozone raccordé dans le flux de l'air ou de l'oxygène.

9. - Dispositif d'atomisation d'un liquide au moyen d'un fluide gazeux sous pression, caractérisé par un corps cylindrique traversé dans toute sa longueur par au moins un canal rectiligne, parallèle à l'axe du corps, par une chambre de turbulence sur la partie frontale du corps, la chambre étant fermée vers l'avant du dispositif par une paroi frontale à faces planes et parallèles comportant une

0096731

ouverture centrale cylindrique de formation de spray dont la génératrice forme avec les deux faces de la paroi un angle de 90$^{\circ}$ sans bords arrondis ni arêtes.

10. - Dispositif selon la revendication 9, caractérisé en ce que la paroi frontale fait partie d'un capuchon vissé sur la partie antérieure du corps, la chambre étant délimitée latéralement par un rebord circulaire.

11. - Dispositif selon la revendication 9 ou 10, caractérisé en ce que le nombre des canaux et leur diamètre ainsi que le diamètre de l'ouverture centrale sont choisis de façon à créer une différence de pression entre la chambre de turbulence et la pression d'alimentation en amont des canaux.

- 1/1 -

0096731

FIG. 1

FIG. 2

FIG. 3

FIG. 4

FIG. 5

## DOCUMENTS CONSIDERES COMME PERTINENTS

| Catégorie | Citation du document avec indication, en cas de besoin, des parties pertinentes | Revendication concernée |
|---|---|---|
| X | CH - A5 - 625 959 (BERNAZ) <br> * Entièrmant * | 1,3-7 |
| | -- | |
| A | FR - A1 - 2 400 370 (SOMATRON) <br> * Page 1, lignes 1-9; page 2, ligne 24 - page 3, ligne 17 * | 1 |
| | -- | |
| A | DE - A1 - 2 411 389 (NEMECTRON) <br> * Page 6, lignes 4-21; fig. 1 * | 1,5 |
| | -- | |
| A | US - A - 4 014 345 (KAMEY) <br> * Abrégé; fig. 3 * | 1 |
| | -- | |
| A | FR - A - 2 140 948 (BERTHOUD) <br> * Fig. 2 * | 2,9-11 |
| | -- | |
| A | DE - A1 - 2 814 246 (METALLGESELL-SCHAFT) <br> * Page 6, lignes 11-22; fig. 1 * | 2,9-11 |
| | -- | |
| A | US - A - 4 260 110 (WERDING) <br> * Abrégé; colonne 1, lignes 7-29; colonne 14, lignes 12-58; fig. 4 * <br> & FR-A-2 371 238 <br> & FR-A-2 399 282 | 2,9-11 |
| | ---- | |

**CLASSEMENT DE LA DEMANDE (Int. Cl.)** 3

A 61 N 1/04
A 61 M 11/00
B 05 B 1/00

**DOMAINES TECHNIQUES RECHERCHES (Int. Cl.)** 3

A 61 N
A 61 M
B 05 B

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

| X | Le présent rapport de recherche a été établi pour toutes les revendications |
|---|---|

| Lieu de la recherche | Date d'achèvement de la recherche | Examinateur |
|---|---|---|
| VIENNE | 23-12-1982 | NEGWER |

OEB Form 1503.1 06.78